Europäisches Patentamt

European Patent Office

Office européen des brevets

(11) Publication number : 0 510 951 A2

# (12) EUROPEAN PATENT APPLICATION

(21) Application number : 92303623.0

(22) Date of filing : 22.04.92

(51) Int. Cl.⁵ : **G01N 1/10, G01N 30/06**

(30) Priority : **22.04.91 JP 116570/91**

(43) Date of publication of application :
**28.10.92 Bulletin 92/44**

(84) Designated Contracting States :
**AT BE CH DE FR GB IT LI LU NL SE**

(71) Applicant : **Japan National Oil Corporation**
**2-2, Uchisaiwaicho 2-chome**
**Chiyoda-ku Tokyo (JP)**

(72) Inventor : **Fukuda, Munehiro**
**405 Als-Shinmatsudo, 10-2 East Shinmatsudo**
**Matsudo City, Chiba Prefecture (JP)**
Inventor : **Hua, Kenka Inn Yin Jian**
**203 2nd Fuji Height, 4-17-7 Sugano**
**Ichikawa City, Chiba Prefecture (JP)**

(74) Representative : **Lyons, Andrew John et al**
**ROYSTONS 531 Tower Building Water Street**
**Liverpool L3 1BA (GB)**

(54) Method for gathering and analysing hydrocarbon contained in sea water in small quantities.

(57)   A prescribed quantity of sea water is passed through one or more adsorption cylinders filled with adsorbent to bring the molecule of hydrocarbon contained in the sea water in small quantities into direct contact with the adsorbent to let the adsorbent adsorb the hydrocarbon. Then, by heating and treating the adsorbent adsorbing the hydrocarbon to be analyzed, even light-duty hydrocarbon having relative large molecular weight and small volatility can be effectively detected.

EP 0 510 951 A2

DESCRIPTION

This invention relates to a method for gathering and analyzing hydrocarbon contained in sea water for the purpose of offshore exploration for natural resources such as oil and gas.

In the course of exploration for underground natural resources, hydrocarbons, particularly $C_5$-$C_8$, serves as an essential sign indicating the existence of oil and natural gas depositing underseas. There has been a system of performing a degassing analysis of sea water while continuously taking in sea water. This system generally called the "Sniffer" was developed by Interocean Systems, Inc. (U.S.A.). In the system, sea water is taken in by a pump and subjected to bubbling treatment by using an inert gas on a survey vessel to dissolved extract light hydrocarbon. Then, the hydrocarbon thus extracted is analyzed by using a gas chromatograph. A similar system has been manufactured by Gulf Oil Company (U.S.A.) and practically used for exploration by Exploration Technologies, Inc. This system can extract hydrocarbon dissolved in sea water by vacuum suction and then analyze the extracted hydrocarbon.

In the aforenoted "Sniffer" system for offshore petroleum exploration, lighter hydrocarbons including up to butane with higher volatility are mainly measured. However, the hydrocarbons with lower volatility, from pentane to octane, can be detected only when it is dissolved with higher concentration. As for aromatic hydrocarbon measured by the "Sniffer", there is no available data for hydrocarbon having low concentration below the order of ppb.

The conventional analyzing systems noted above utilize the volatility of hydrocarbon to extract the hydrocarbon from sea water. This method is applicable to lighter hydrocarbons such as methane, ethane, propane and butane, but unsuitable for hydrocarbons being higher molecular weight such as pentane, hexane, heptane, octane benzene, toluene, ethylbenzene, m-xylene, p-xylene and o-xylene, because of lower volatility and generally lower concentration in sea water which make both extraction and analysis difficult. Aromatic hydrocarbon in sea water such as benzene, toluene, ethylbenzene and xylene in particular could not be analyzed by the conventional analyzing method, unless it is extremely high in concentration. Since the content of these hydrocarbons can also be significant for offshore exploration of natural resources such as oil and gas, there has been a great need for a useful method for gathering and analyzing hydrocarbon in sea water.

An object of the present invention is to provide an effective method for gathering and analyzing hydrocarbon which is dissolved in sea water in small quantities including those with relatively higher molecular weight, so as to obtain a significant sign definitely indicating offshore underground resources such as oil and natural gas.

To attain the object mentioned above according to the present invention, there is provided an adsorption method to gather hydrocarbon dissolved in sea water. Sea water is introduced into an adsorption cylinder filled with adsorbent to allow the the hydrocarbon molecule contained in sea water in small quantities to contact directly with the surface of the adsorbent, thereby permitting the hydrocarbon having various molecular weights to be adsorbed regardless of volatility.

The adsorbent may be of any type, only if it can selectively adsorb the hydrocarbon to be measured, however, molecular sieve composed of carbon, and graphited carbon black are preferable. According to the present method, even hydrocarbon dissolved in sea water in small quantities on the order of ppb can be effectively gathered and analyzed, and particularly, aromatic hydrocarbon such as benzene and toluene can be measured very accurately.

Other and further objects of this invention will become obvious upon an understanding of the illustrative embodiments about to be described or will be indicated in the appended claims, and various advantages not referred to herein will occur to one skilled in the art upon employment of the invention in practice.

The other objects and features of the present invention will be hereinafter explained in detail with reference to the accompanying drawings, wherein:

FIG. 1A is a block diagram showing a primary adsorbing process in one embodiment of the gathering and analyzing method according to this invention;

FIG. 1B is a block diagram showing a primary desorbing and secondary adsorbing processes of the same;

FIG. 1C is a block diagram showing a secondary desorbing and gas-chromatographic analyzing processes of the same; and

FIG. 2 is a graph showing the results (ionic strength) of analysis for benzene and toluene in the sea water taken in, according to this invention.

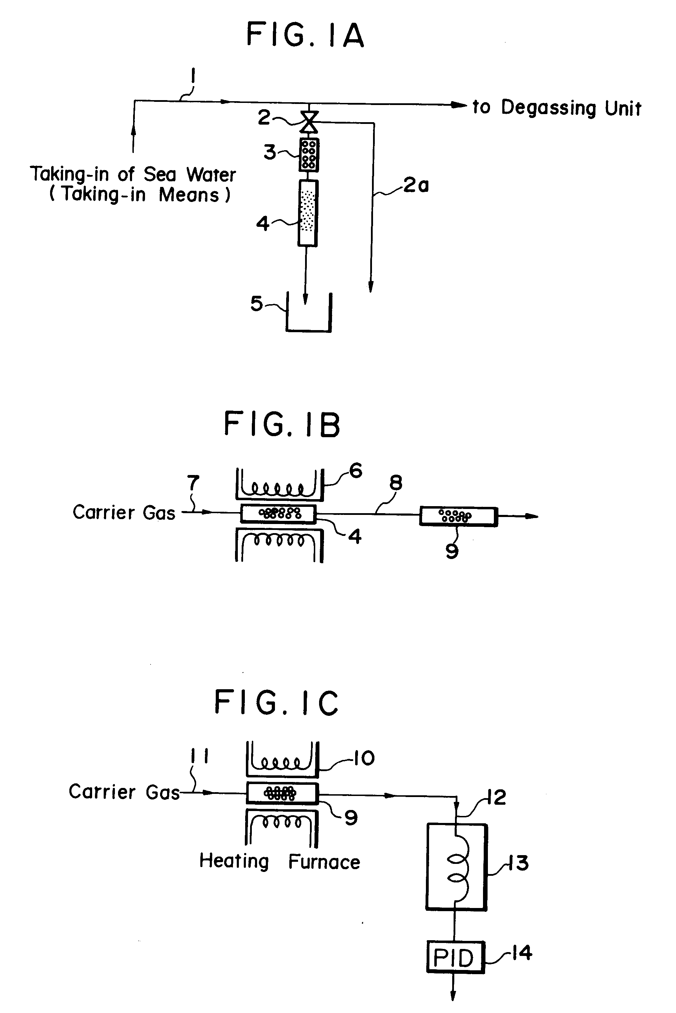

The present invention will become more fully understood from the detailed description given hereinbelow and the accompanying drawings which are given by way of illustration only, and thus are not limitative of the present invention, and wherein FIG. 1A and FIG. 1B show a system used for gathering and analyzing hydrocarbon existed in sea water in extremely small quantities according to the present invention.

In FIG. 1A, a sea water sample is introduced into a primary adsorption cylinder 4 through a pipe 1, a valve 2 and a filter 3 for removing suspended matter in the sea water. The primary adsorption cylinder 4 is detachable from the system and filled with adsorbent as will be described later in detail. The sea water passed through the primary adsorption cylinder 4 is then received by a quantitative receptacle 5 for measuring the quantity of the sea water. When the quantity of the sea water flowing out from the adsorption cylinder 4 reaches a prescribed value, the valve 2 is changed over to a distributing pipe 2a for draining, so that the adsorption cylinder 4 can be replaced.

The primary adsorption cylinder 4 through which a predetermined quantity of sea water passed is dealt with by a centrifugal separator to sufficiently remove water from inside the adsorption cylinder 4. After dehydrating, the adsorption cylinder 4 is detached from the centrifugal separator and set in a heating furnace 6. The adsorption cylinder 4 set in the furnace 6 is connected at its one end to a carrier gas supplying pipe 7 and at the other end thereof to a pipe 8 leading to a secondary adsorption cylinder 9. The adsorbent in the adsorption cylinder 4 is heated at an optimum temperature, for example, 200°C to 400°C, by the heating furnace 6 with flowing carrier gas through inside the adsorption cylinder 4. Thus, components which are adsorbed by the adsorbent by passing the sea water through the primary adsorption cylinder 4 are discharged and sent to the secondary adsorption cylinder 9 together with the carrier gas, thereby to be adsorbed to adsorbent contained in the adsorption cylinder 9. In the case of heating at the aforenoted temperature, 90% or more hydrocarbon such as benzene is held adsorbed in the secondary adsorption cylinder 9.

The secondary adsorption cylinder 9 having the adsorbent dehydrated is then set in the heating furnace 10 as shown in FIG. 1C and connected at its one end to a carrier gas supplying pipe 11 and at the other end thereof to a pipe 12 leading to a gas-chromatograph column 13. The adsorbent in the adsorption cylinder 9 is heated at, for example, 200°C to 400°C, by the heating furnace 10 while flowing carrier gas through inside the adsorption cylinder 9. Thus, components which are adsorbed to the adsorbent in the adsorption cylinder 9 are discharged and sent to the column 13 together with the carrier gas, thereby to be separated by gas-chromatograph. The reference numeral 14 [PID] denotes a detector connected to the outlet side of the column 13.

As the adsorbent contained in the adsorption cylinders, molecular sieve composed of carbon, graphited carbon black or the like can be preferably used. As one example, the molecular sieve is on the market in the trade name of "Carboxen 56411 and "Carbosieve S-III", and the graphited carbon black is on the market in the trade name of "Carbotrap".

In the system illustrated in FIGS. 1A through 1C, the elements of the following types were used actually for practicing the method of this invention.

[Primary Adsorption Cylinder]

| | |
|---|---|
| Inner diameter | : 8 mm |
| Length | : 12 cm |
| Adsorbent | : Carboxen 564 |

[Secondary Adsorption Cylinder]

| | |
|---|---|
| Inner diameter | : 2 mm |
| Length | : 11.5 cm |
| Adsorbent | : Carbotrap (Front half part) |
| | Carbosieve S-III (Rear half part) |

In the experiment actually conducted, the valve 2 shown in FIG. 1A was changed over each time 2000 ml of the sea water flows into the primary adsorption cylinder 4, and then, the primary adsorption cylinder 4 was replaced. The replacement of the primary adsorption cylinders 4 was carried out every 10 minutes. Then, each primary adsorption cylinder 4 was set in the heating furnace 6 after dehydrating by the centrifugal separator, as illustrated in FIG. 1B, and there heated at 230°C for 5 minutes while flowing $N_2$ as carrier gas through the primary adsorption cylinder 4 and secondary adsorption cylinder 9 at a rate of 60 ml/min. Thereafter, the secondary adsorption cylinder 9 was set in the heating furnace 6 as shown in FIG. 1C and heated at a relatively low temperature (100°C to 120°C) for 2 minutes while being supplied with carrier gas such as $N_2$ and an inert gas like He, so as to remove sufficiently the moisture which could not be removed by the centrifugal separator.

Next, the secondary adsorption cylinder 9 was displaced from the furnace 6 to the heating furnace 10 as shown in FIG. 1C, and then, connected at its one end to an N gas supply source (not shown) for supplying the carrier gas and at the other end thereof to the gas-chromatograph column 13. In this state, the secondary adsorption cylinder 9 was heated at 290°C for 3 minutes while flowing $N_2$ gas at a rate of 15 ml/min.

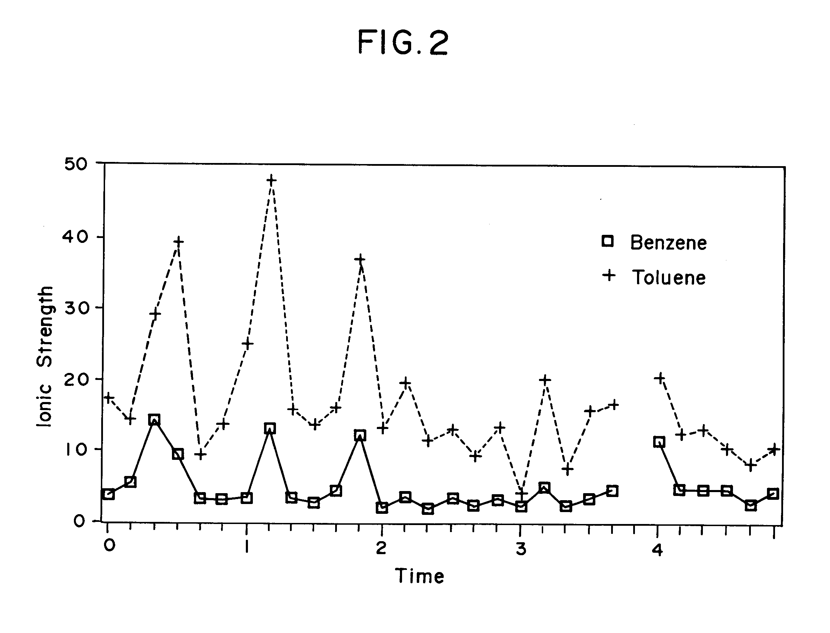

The sea water samples supplied for the analysis were gathered off the coast of Niigata in the Japan Sea from the survey vessel, and could be confirmed to contain benzene and toluene as shown in FIG. 2. To be more specific, the sea water samples were continuously gathered from the moving vessel within the specified sea area off Niigata at the fixed time intervals of 10 minutes so as to measure the relative concentrations (ionic strength) of benzene, toluene, ethylbenzene, m-xylene, p-xylene and o-xylene contained in each sea water gathered at the respective points.

As is plain from the foregoing description, according to the present invention, hydrocarbon dissolved in sea water can be effectively analyzed even if it has relatively higher molecular weight and small volatility. Therefore, a significant sign definitely indicating underground natural resources can be readily and accurately obtained during offshore exploration.

Although the invention has been described in its preferred form with a certain degree of particularity, it is understood that the present disclosure of the preferred form has been changed in the details of construction and the combination and arrangement of parts may be resorted to without departing from the spirit and the scope of the invention as hereinafter claimed.

## Claims

1. A method for gathering and analysing hydrocarbon contained in sea water in small quantities, which comprises passing a prescribed amount of sea water sample through a primary adsorption cylinder filled with adsorbent, heating said primary adsorption cylinder while flowing carrier gas through inside said primary adsorption cylinder so as to let the adsorbent desorb hydrocarbon with said carrier gas, passing said carrier gas containing hydrocarbon through a secondary adsorption cylinder filled with adsorbent, heating siad secondary adsorption cylinder while flowing carrier gas through inside said secondary adsorption cylinder so as to let the adsorbent desorb hydrocarbon with said carrier gas, and analysing said carrier gas containing hydrocarbon.

2. A method as claimed in claim 1, wherein one or both of said primary and secondary adsorption cylinders are subjected to centrifuging so as to dehydrate the adsorbent contained therein upon completion of adsorption.

3. A method as claimed in claim 1 or 2, wherein said secondary adsorption cylinder is heated at 100°C to 120°C for about 2 minutes while being supplied with carrier gas so as to dehydrate the adsorbent contained therein.

4. A method as claimed in claim 1, 2 or 3, wherein the adsorbent is a molecular sieve of carbon or graphited carbon black.

5. A method as claimed in any one of claims 1 to 4, wherein the primary adsorption cylinder is heated to 200 to 400°C.

6. A method as claimed in any one of claims 1, 2, 4 and 5, wherein the secondary adsorption cylinder is heated to 200 to 400°C.

7. A method as claimed in any one of claims 1 to 6, wherein the carrier gas is nitrogen.

# FIG. IA

to Degassing Unit

Taking-in of Sea Water
( Taking-in Means )

# FIG. IB

Carrier Gas

# FIG. IC

Carrier Gas

Heating Furnace

PID

# FIG.2

EP 0 510 951 A2